# EUROPÄISCHE PATENTSCHRIFT

(11) **EP 1 690 506 B1**
(45) Veröffentlichungstag und Bekanntmachung des Hinweises auf die Patenterteilung: **20.08.2008**
(21) Anmeldenummer: 05100927.2
(22) Anmeldetag: 09.02.2005
(51) Int. Cl.: A61B 17/66

(54) **Externe Befestigungsvorrichtung, insbesondere zum Vergrössern eines Abstandes zwischen Klemmelementen**
External fixation device; in particular for increasing a distance between clamping elements
Dispositif externe de fixation, notamment pour agrandir une distance entre des éléments de serrage

(43) Veröffentlichungstag der Anmeldung: 16.08.2006
(73) Patentinhaber: Stryker Trauma SA, 2545 Selzach (CH)
(72) Erfinder: Baumgartner, Thomas, 4552, Derendingen (CH); Bernhard, Andreas, 2554, Meinisberg (CH)
(74) Vertreter: Liebetanz, Michael

(56) Entgegenhaltungen:
- EP-A- 0 385 929
- EP-A- 0 566 561
- DE-U1- 9 214 550
- US-A1- 2004 059 331

## Beschreibung

### Technisches Gebiet der Erfindung:

Die Erfindung betrifft eine externe Befestigungsvorrichtung, insbesondere zum Vergrössern eines Abstandes zwischen Klemmelementen. Sie betrifft weiterhin eine solche Befestigungsvorrichtung für die Osteosynthese und hierbei beispielsweise zum Einsatz für einen externen Fixator.

Sie betrifft in anderen Worten eine externe Befestigungsvorrichtung, insbesondere zum Vergrössern eines Abstandes zwischen Klemmelementen für einen externen Fixator, mit einem zentralen Innenschaft, mit einer äusseren hohlen Hülse, in der der Innenschaft axial beweglich ist, wobei ein Ende des Innenschaftes über das eine Ende der Hülse hinausragt, und mit einem Einstellelement, das an dem anderen Ende der Hülse vorgesehen ist und mit dem inneren Schaft und/oder der äusseren hohlen Hülse verbunden ist, wobei durch eine Drehung des Einstellelementes die axiale Position des Innenschaftes in Bezug auf die Hülse einstellbar ist.

### Stand der Technik:

Eine solche Vorrichtung ist aus der FR 2,557,933 bekannt. Sie wird insbesondere im Rahmen eines externen Fixateurs eingesetzt. Sie umfasst einen rechteckigen Innenschaft und ein diesen umgebendes Hohlrohr, die somit verdrehsicher ineinander schiebbar sind. Die Einstellung soll über eine Mutter und Einstellschraube erfolgen, die an dem freien Ende des Hohlrohrs vorgesehen sind. An den beiden Enden der Vorrichtung ist oder wird ein Klemmelement befestigt, welches über Stäbe oder Stifte mit Knochenteilen verbunden werden kann. An dem Hohlrohr ist ein Klemmelement über einen das Rohr umfassenden Bügel befestigt, das somit gegenüber der Achse des rechteckigen Rohrs verdrehsicher ist. An dem freien Ende des Innenschaftes ist ein weiteres Klemmelement durch eine longitudinal in den Innenschaft einschraubbare Schraube befestigt, das somit gegenüber der Achse des rechteckigen Innenschaftes frei verdrehbar ist. Als Material werden für den einmaligen Gebrauch nicht metallische Materialien genannt. Nach Fixieren von zwei gegenüberliegenden Knochenstücken mit zwei Klemmelementen, ist es somit durch Betätigen der Einstellvorrichtung, das heisst Mutter und Einstellschraube, möglich, den Abstand der Befestigungspunkte des Fixators im Knochen voneinander zu vergrössern, das heisst die Länge des Knochens über alles zu vergrössern.

Aus der US 5,454,810 ist eine weitere solche Vorrichtung bekannt. Diese Druckschrift nennt das Problem des Einsatzes eines rotationssymmetrischen Innenschaftes und Aussenrohrs in Bezug auf die rotatorische Stabilität. Bei der US 5,454,810 wird ein rundes Aussenrohr mit einer durchgehenden im Querschnitt quadratischen Ausnehmung eingesetzt, so dass ein im Querschnitt quadratischer Innenschaft in das Aussenrohr einsetzbar ist. Das Aussenrohr ist aus zwei Aluminiumhälften aufgebaut, während der Innenschaft aus Stahl besteht. Um Verklemmungen zu vermeiden, nutzt der Stand der Technik in die Ausnehmung eingesetzte Polymermaterialstreifen.

Das freie Ende des Innenschaftes endet in einem aus dem Aussenrohr herausragendem Teil mit grösserem Durchmesser. Dieses Ende dient zur Befestigung des einen Klemmelementes. Das andere Ende des Innenschaftes stösst bei der teleskopierbaren Version der Vorrichtung nach US 5,454,810, dargestellt mit Elementen in der dortigen Fig. 8, gegen einen durch ein Schraubgewinde axial beweglichen Stössel.

EP 0 566 561 zeigt eine externe Fixierungsvorrichtung für Knochen, welche sowohl für Bewegung von Knochen als auch für verlängerungsverfahren von Knochen geeignet ist. Die Fixierungsvorrichtung umfasst einen nicht-rotierbaren, steifen Schaft, welcher entlang einer longitudinalen Achse bewegbar ist.

Diese Vorrichtungen weisen den Nachteil auf, dass sie schwierig zu justieren sind, denn beide Druckschriften verweisen auf den die Einstellungen vornehmenden operierenden Chirurg. Die an dem freien Ende bestehenden Befestigungsknöpfe können dagegen auch durch den Patienten, sowohl im Krankenhaus als auch bei ambulanter Behandlung, unbeabsichtigt betätigt und somit verstellt werden.

### Zusammenfassung der Erfindung:

Ein Nachteil der bestehenden Vorrichtungen dieser Art liegt also insbesondere daran, dass sie nicht einfach benutzbar sind. Sie sollten vorteilhafterweise auch vom Benutzer selber definiert veränderbar sein, beispielsweise sollte ein Patient den besagten Abstand, das heisst die besagte Knochenlänge, auf einfachste Weise selbst vergrössern können. Gleichzeitig sollte die Vorrichtung gegen unbeabsichtigte Betätigung gesichert sein.

Ausgehend von diesem Stand der Technik liegt der Erfindung also die Aufgabe zugrunde, eine Vorrichtung der eingangs genannten Art so zu verbessern, dass die Vorrichtung einfacher, insbesondere vom Patienten selber, definiert verstellbar ist. Gleichzeitig soll sie besser gegen unbeabsichtigtes Verstellen geschützt sein.

Diese Aufgabe wird erfindungsgemäss mit den Merkmalen des Anspruchs 1 dadurch gelöst, dass Einstellknopf und Hülse über jeweils mindestens ein komplementäres Rastelement verfügen, so dass durch Anzahl und Anordnung der komplementären Rastelemente nach jeweils einem vordefinierten Drehwinkel des Einstellknopfes eine Rastposition mit einer geänderten Länge erreichbar ist.

Unter Verrastung ist hier kein absolutes Festlegen im Sinne einer Verriegelung der Vorrichtung verstanden, sondern eine durch geringe Kraft überwindbare die Drehung hindernde Feststellung, die einem die Drehung ausführenden Benutzer ein taktiles Erlebnis ermöglicht. Das eine Ende des Innenschaftes ragt über das eine Ende der Hülse hinaus und dient dabei zur Befestigung des einen Klemmelementes, während die Hülse zur Befestigung des anderen Klemmelementes dient. Eine Drehung in die eine Richtung führt zu einer Vergrösserung in der geänderten Länge, eine Drehung in die andere Richtung einer Verkleinerung. Beide Änderungen sind möglich.

Dadurch, dass der Einstellknopf wieder abgenommen werden kann, wird die Vorrichtung insgesamt kleiner. Das macht es für den Patienten leichter, die Vorrichtung zur Verlängerung des Knochenabstandes periodisch, insbesondere täglich, durchzuführen.

Vorteilhafterweise ist eine Aufhängeeinrichtung beispielsweise in Form einer Öse vorgesehen.

Die Aufgabe wird in Bezug auf das Lösen der Vorrichtung im Ganzen mit den Merkmalen des Anspruchs 6 dadurch gelöst, dass das Einstellelement einen Schaft mit einem Aussengewinde aufweist, dass der Innenschaft mindestens teilweise hohl ist und an seinem hohlen Ende über ein Innengewinde verfügt, in dass das besagte Aussengewinde eingreift, dass Innenschaft und Hülse einen zueinander komplementären verdrehsicheren Querschnitt aufweisen, dass das Einstellelement und die Hülse über komplementäre nachgiebige Rastmittel verfügen, um die mit dem Innenschaft verbundenen Einstellmittel und die Hülse gegenüber einer direkten axialen Verschiebung zueinander zu verriegeln.

Unter Verriegeln ist hier ein Festlegen der Vorrichtung zu verstehen, die eine Verkürzung des Abstandes der Klemmelemente voneinander vermeidet.

Weitere vorteilhafte Ausführungsformen sind in den Unteransprüchen gekennzeichnet.

### Kurze Beschreibung der Zeichnungen:

Ein Ausführungsbeispiel der Erfindung wird nun beispielhaft an Hand der Zeichnungen näher erläutert. Es zeigen:
- Fig. 1: eine perspektivische Darstellung einer Vorrichtung gemäss einem Ausführungsbeispiel der Erfindung mit einem an dieser befestigten Klemmelement,
- Fig. 2: eine teilweise Querschnittsansicht der Vorrichtung nach Fig. 1,
- Fig. 3: eine teilweise Querschnittsansicht der Vorrichtung nach Fig. 1 ohne externes Klemmelement,
- Fig. 4: eine vergrösserte Schnittansicht des einen Endes der Hülse,
- Fig. 5: eine perspektivische Ansicht des Innenschaftes,
- Fig. 6: eine Querschnittsansicht des Innenschaftes nach den Linien VI-VI der Fig. 5,
- Fig. 7: eine Seitenansicht des Einstellelementes,
- Fig. 8: eine perspektivische Ansicht des Einstellknopfes,
- Fig. 9: eine teilweise Querschnittsansicht der Vorrichtung nach Fig. 1 mit Einstellknopf nach Fig. 8,
- Fig. 10: eine Detailansicht der Umlaufnut des Einstellknopfes,
- Fig. 11: eine erste perspektivische Ansicht eines Rastelementes des Einstellknopfes, und
- Fig. 12: eine zweite perspektivische Ansicht eines Rastelementes des Einstellknopfes.

### Ausführliche Beschreibung eines Ausführungsbeispiels:

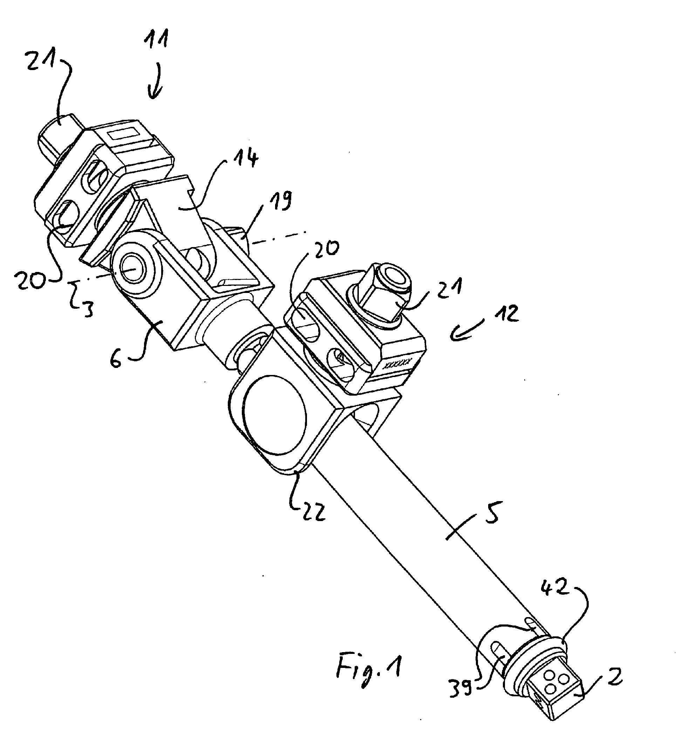

Die Fig. 1 zeigt eine perspektivische Darstellung einer externen Befestigungsvorrichtung 1 gemäss einem Ausführungsbeispiel der Erfindung mit einem an dieser befestigten externen zweiten Klemmelement 12. An diesem externen zweiten Klemmelement 12 ist beim Einsatz der Vorrichtung ein in den Zeichnungen nicht dargestelltes stabförmiges Element befestigt, welches in einem Knochenteil befestigt ist. Mit dem Bezugszeichen 11 ist ein im wesentlichen in Verlängerung der Vorrichtung 1 angeordnetes erstes Klemmelement bezeichnet, an dem ebenfalls ein weiteres stabförmiges Element befestigbar ist. Dieses weitere stabförmige Element wird beim Einsatz der Vorrichtung an einem anderen Knochenteil befestigt, beispielsweise derart, dass zwischen dem oben genannten und diesem Knochenteil eine Bruchstelle liegt, deren Abstand mit dieser Vorrichtung vergrösserbar ist.

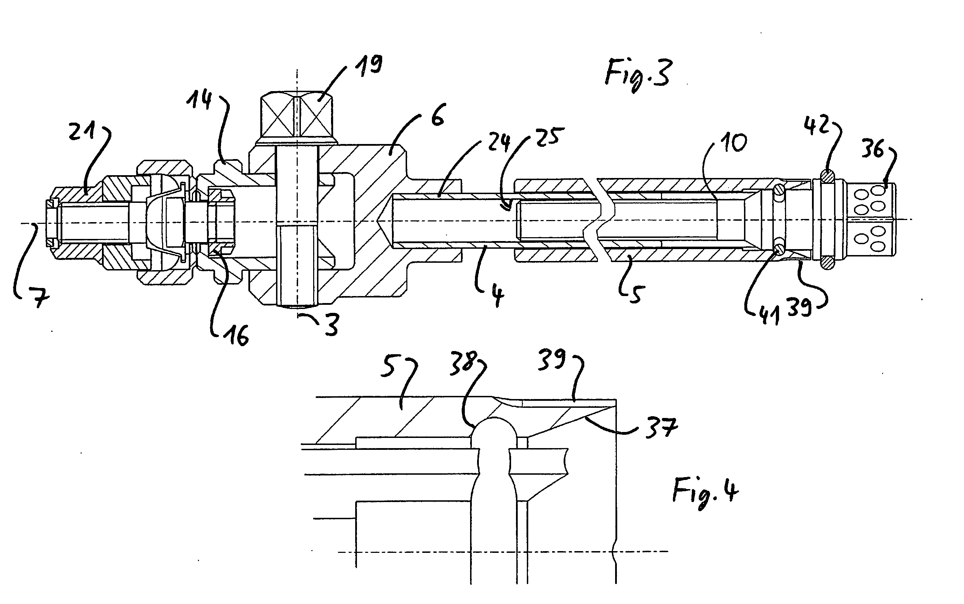

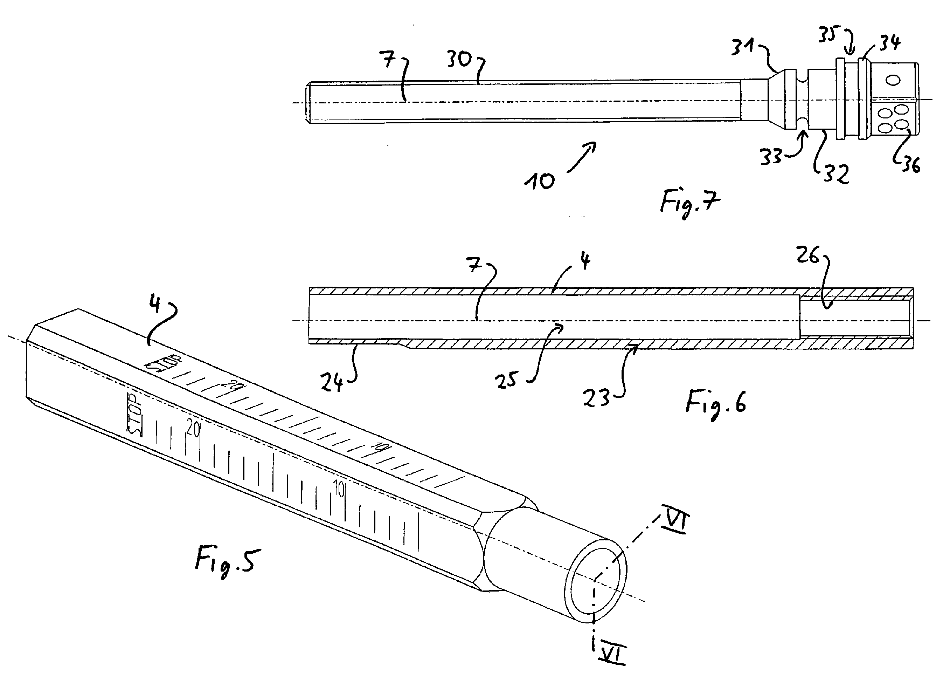

Eine solche externe Befestigungsvorrichtung 1 zum Vergrössern eines Abstandes zwischen Klemmelementen 11, 12 für einen externen Fixator verfügt über einen zentralen Innenschaft 4, der erst in der Fig. 3 richtig sichtbar ist, da er in einer äusseren hohlen Hülse 5 der Vorrichtung 1 angeordnet ist. In dieser Hülse 5 ist der Innenschaft 4 indirekt über ein erst in Fig. 3 dargestelltes Einstellelement 10 axial beweglich. Dabei ragt ein Ende des Innenschaftes 4 über das eine Ende der Hülse 5 hinaus und ist in dem dargestellten Ausführungsbeispiel mit einem Bügel 6 verbunden, an dem das erste Klemmelement 11 verschwenkbar befestigt ist. Die Vorrichtung 1 verfügt ferner über ein Einstellelement 10, das in der Fig. 7 dargestellt ist und das auf das andere Ende der Hülse 5 aufsetzbar ist, wobei ein im Querschnitt quadratischer Eingriffkopf 36 vorgesehen ist, dessen Funktion in den nachfolgenden Zeichnungen noch näher beschrieben wird. Mit dem Einstellelement ist die axiale Position des Innenschaftes 4 in Bezug auf die Hülse 5 einstellbar.

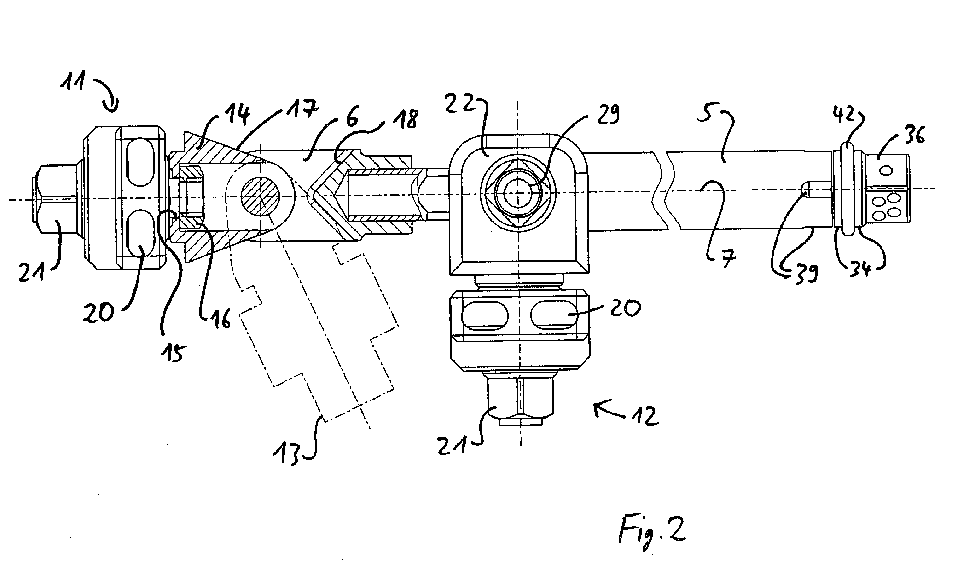

Das erste Klemmelement 11 gehört beim dargestellten Ausführungsbeispiel integral zur Vorrichtung 1 und ist um eine Achse 3 verschwenkbar mit der Vorrichtung 1 verbunden. In der Fig. 2 ist eine teilweise Querschnittsansicht der Vorrichtung nach Fig. 1 dargestellt. Gleiche Merkmale sind in allen Zeichnungen mit den gleichen Bezugszeichen versehen. Das erste Klemmelement 11 ist um die besagte Achse 3 verschwenkbar, beispielsweise um über 90 Grad, beispielsweise 115 Grad wie hier, bis in die strichliniert dargestellte Lage 13. An der Achse 3 ist ein Hohlkörper 14 befestigt, in der in dessen radialer Richtung eine Bohrung 15 vorgesehen ist, in der das erste Klemmelement 11 einsetzbar und verklemmbar ist. Dafür kann eine Schraube 16 in die Bohrung 15 eingesetzt werden. Der Hohlkörper 14 verfügt über seitliche Anschlagkanten 17, die mit entsprechenden Anschlagkanten 18 des Bügels 6 die Verschwenkbewegung des Klemmelementes 11 um die Achse 3 begrenzen. Der Hohlkörper 14 wird über eine in den Bügel 6 eingreifende Klemmeinrichtung 19, beispielsweise eine Schraube, in einer definierten Winkelposition fixiert.

In anderen in den Zeichnungen nicht dargestellten Ausführungsbeispielen kann die Verlängerung des Innenschaftes 4 ein zylindrisches Element mit dem Querschnitt der Hülse 5 aufweisen. Damit ist es möglich anstelle des integrierten ersten Klemmelementes 11 ein weiteres zweites Klemmelement 12, wie es nachfolgend beschrieben wird, als "erstes" Klemmelement einzusetzen.

Das zweite externe Klemmelement 12 ist an der Hülse 5 der Vorrichtung 1 über einen Bügel 22 befestigt. Die Schenkel des die Hülse 5 umgreifenden Bügels werden durch eine Klemmschraube 29 gegen die Hülse verspannt. In der Hülse 5 verläuft der Schaft 4. Durch die im Nachhinein beschriebenen Elemente 2, 10 ist der Schaft 4 gegenüber der Hülse 5 longitudinal verschiebbar, so dass sich das erste Klemmelement 11 gegenüber dem zweiten Klemmelement 12 bewegen lässt. Dabei führen die Klemmelemente 11 und 12 eine translatorische Bewegung zueinander aus, auch wenn die Achsen von von den Klemmelementen 11, 12 geklemmten Stäben nicht parallel zueinander ausgerichtet sein müssen. Bei den hier beispielhaft dargestellten Klemmelementen 11 und 12 werden die Stäbe jeweils in die Öffnungen 20 eingesetzt und durch die Klemmschrauben 21 festgesetzt.

Die Fig. 3 zeigt eine teilweise Querschnittsansicht der Vorrichtung 1 nach Fig. 1 ohne externes Klemmelement 12. Das Klemmelement 21 fixiert im Zusammenspiel mit der Schraube 16 das Klemmelement 11. Die Funktion der Klemmschraube 19 ist sehr einfach zu beschreiben, da sie das den Bügel 6 zusammendrückt und damit den Hohlkörper 14 festklemmt und festlegt.

Die folgende Beschreibung ist im Zusammenhang mit der Fig. 4, die eine vergrösserte Schnittansicht des einen Endes der Hülse 5 zeigt, mit der Fig. 5, die eine perspektivische Ansicht des Innenschaftes 4 zeigt, mit der Fig. 6, die eine Querschnittsansicht des Innenschaftes 4 nach den Linien VI-VI der Fig. 5 zeigt, und mit der Fig. 7 zu sehen, die eine Seitenansicht des Einstellelementes 10 zeigt.

Die Hülse 5 verfügt über eine im wesentlichen quadratische Innenausnehmung, die komplementär zum Aussenmantel 23 des Innenschaftes 4 ausgestaltet ist. Der Aussenmantel 23 ist an seinen flachen Seiten jeweils mit einer Ableseskala versehen. Dabei ist der Wert jeweils des Skalenstriches abzulesen, der über die Hülse 5 herausragt. Der Innenschaft 4 ist an seinem Ende in einen Zylinder 24 abgedreht, so dass er in dem Bügel 6 befestigbar ist. Der in Fig. 3 dargestellte Schnitt entspricht einer Draufsicht auf eine rechteckige Seite des Innenschaftes 4.

Der Innenschaft 4 ist hohl ausgestaltet. Die zylindrische Innenbohrung 25 ist vorzugsweise durchgehend, könnte aber auch im Bereich des abgedrehten Zylinders 24 als Sackloch enden. An dem dem Zylinder 24 gegenüberliegenden Ende ist der Innenschaft 4 innenseitig mit einem Innengewinde 26 ausgestaltet, dessen Aussendurchmesser mindestens nicht grösser als der Innendurchmesser der Innenbohrung 25 ist, so dass das Einstellelement 10 über das Gewinde 26 in den Schaft 4 einschiebbar ist.

Das Einstellelement 10 verfügt über einen gewindeten Schaft 30 ausreichender Länge, wobei die Länge, zusammen mit den Massen der Hülse 5 und des Innenschaftes 4 den Hub der Teleskopfunktion festlegt. Das Gewinde 26 und der komplementäre gewindeten Schaft 30 sind vorteilhafterweise mit einem sehr feinen Gewinde ausgestaltet, so dass eine Vielzahl von Drehungen notwendig sind, um einen gewissen translatorischen Hub zu erreichen.

An diesen gewindeten Schaft 30 schliesst sich nach einem kurzen ungewindeten Abschnitt eine konische Aufweitung 31 an, die in einen einen grösseren Durchmesser aufweisenden zylindrischen Abschnitt 32 übergeht, in dem eine kreisförmig umlaufende Nut 33 vorgesehen ist. Flanschartig sich verbreiternd schliesst sich ein weiterer einen grösseren Durchmesser aufweisender zylindrischer Abschnitt 34 an, in dem eine weitere Nut 35 vorgesehen ist. Schliesslich schliesst sich an diesen Abschnitt 34 der Eingriffknopf 36 an, der in der Draufsicht entlang der Achse 7 einen quadratischen Querschnitt hat. Jede Seite des Knopfes 36 hat eine Kodierung an würfelaugenartigen Vertiefungen mit einer Anzahl von eins bis vier. Damit kann ein Gabelschlüssel eingesetzt werden, und das Weiterdrehen der Augen als jeweils eine Viertelumdrehung gezählt werden. Auch kann der Benutzer durch die in einer Drehrichtung ansteigende Augenzahl die Drehrichtung für die Verlängerungsfunktion erkennen.

Die Fig. 3 zeigt, dass in die Nut 33 ein Klemmring 41 eingelegt wird, der durch die Aufgleitkanten 37 in die Innennut 38 der Hülse 5 eingesetzt wird. Die weiteren Linien in Fig. 4 gehören zur Vierkantaufnahme. Nach dem Einsetzen des Klemmrings 41 in der Nut 38 in der Hülse stösst auch die Schulter zwischen den Abschnitten 32 und 34 nahe an die Hülse 5. In der Nut 35 in dem Abschnitt 34 ist der erste O-Ring 42 eingesetzt. Auf der Aussenseite der Hülse 5 sind in einem regelmässigen Winkelabstand von 90 Grad jeweils eine Laufflächennut 39 abgefast, bei der es sich um eine auch in Umkreisrichtung der Hülse 5 abgerundete Vertiefung handelt, deren Funktion im Zusammenhang mit den Rastelementen 8 deutlich werden wird. Der Klemmring 41 kann beispielsweise eine Stahlfeder sein, die die Elemente 5 und 10 in einem festen Abstand voneinander hält. Damit wird durch Drehen des Elementes 36 lediglich der Innenschaft 4 axial bewegt.

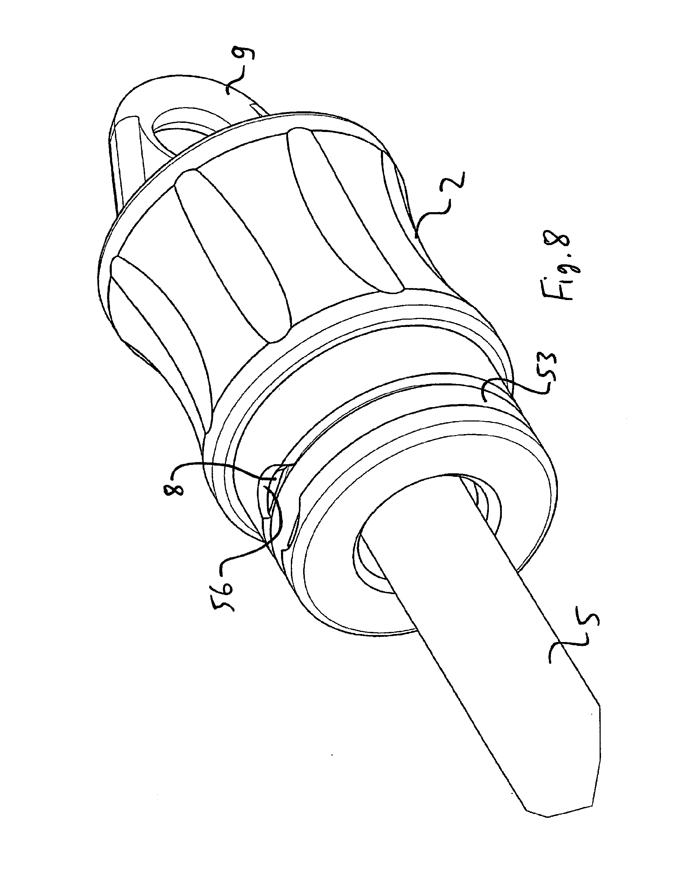

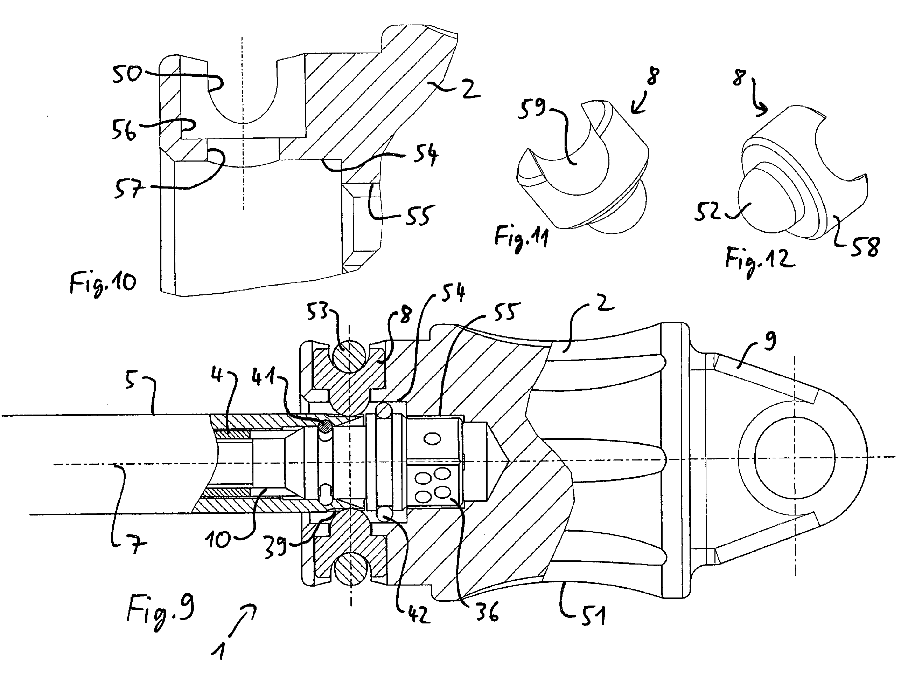

Die Fig. 8 zeigt eine perspektivische Ansicht des Einstellknopfes 2. Die Fig. 9 zeigt eine teilweise Querschnittsansicht der Vorrichtung 1 nach Fig. 1 mit dem Einstellknopf 2 nach Fig. 8. Die Fig. 10 zeigt eine Detailansicht der Umlaufnut 50 des Einstellknopfes 2. Die Fig. 11 und 12 zeigen zwei perspektivische Ansichten des Rastelementes 8 des Einstellknopfes 2. Im Zusammenhang mit der Beschreibung zu diesen Figuren wird auch die Funktionsweise der Vorrichtung erläutert werden.

Auf der linken Bildseite der Fig. 9 ist die Hülse 5 zu erkennen, in der der Innenschaft 4 und das Einstellelement 10 verlaufen.

Der Einstellknopf 2 hat einen Griffbereich 51 für einen Benutzer. An seinem eingriffseitigen Ende verfügt er über eine zweistufige Innenbohrung 54 und Innenvierkant 55 zur Aufnahme des ersten O-Ringes 42 beziehungsweise des Eingriffknopfes 36. Wesentlich ist der O-Ring 42 zum Halten der Klemmeinrichtung 12, die somit auf der Hülse 5 angeordnet werden kann und bei Manipulation der Vorrichtung unverlierbar ist.

In dem Eingriffknopf 2 ist aussen zu der Innenbohrung 54 die Umlaufnut 50 vorgesehen, die an zwei gegenüberliegenden Stellen über eine radial verlaufende zweistufige Bohrung 56 und 57 verfügt. Die erste Stufe 56 verbreitert und vertieft die Umlaufnut 50. Die zweite Stufe 57 bildet eine Durchbohrung zu der Innenbohrung 54. In der Umlaufnut 50 ist ein zweite O-Ring 53 eingesetzt. Zuvor jedoch sind in die besagten Bohrungen 56, 57 Rastelemente 8 eingesetzt. Diese verfügen nach Fig. 11 und 12 über einen zylindrischen oberen Bereich 58 und einen unteren halbkugeligen Knopf 52 geringeren Durchmessers. Dabei passt der zylindrische obere Bereich 58 in die erste Stufe der Bohrung 56 und der Knopf 52 kann durch die zweite Stufe der Bohrung hindurchragen. Durch den O-Ring 53 werden die Rastelemente 8 in ihren Aufnahmen gehalten und gegen die Schulter zwischen den unterschiedliche Durchmesser aufweisenden Abschnitte 56 und 57 gepresst. Dafür weisen die Rastelemente 8 eine quer verlaufende Nut 59 auf, durch die der O-Ring 53 verläuft. Anstelle von zwei Rastelementen 8 können beispielsweise drei im Winkelabstand von 120 Grad oder auch vier im 90 Grad Abstand vorgesehen sein. Auch andere Anzahlen und Winkelverteilungen sind möglich.

Anstelle der getrennten und durch den zweiten O-Ring 53 gehaltenen Bolzen-ähnlichen Rastelemente 8 kann der Einstellknopf 2 auch über Zungen mit den halbkugeligen Knöpfen 52 entsprechenden Ansätzen verfügen, die in die Laufflächennuten 39 eingreifen, so dass der Benutzer ein taktiles Erlebnis hat. Andersherum könnte auch die Hülse 5 mit einem oder mehreren Erhebungen versehen sein, die entsprechend nachgiebige hohlzylindrische Segmente des Einstellknopfes 2 auseinander drücken, wobei dann Nuten im Einstellknopf 2 eine spürbare Verrastung liefern. Es ist auch möglich anstelle der Rastelemente 8 mit halbkugeligen Köpfen 39 direkt einzelne Kugeln einzusetzen, die in dem durch die Umrandungen 56 gebildeten Käfig laufen.

Die Benutzung der Vorrichtung geschieht in zwei Stufen. Der operierende Arzt setzt den externen Fixator mit entsprechenden Befestigungen von stabförmigen Elementen in den gebrochenen Knochen, entweder direkt an der Vorrichtung 1 oder indirekt zu ihr. Dabei setzt er den Einstellknopf 2 auf den Eingriffknopf 36 auf. Beim Aufsetzen des Einstellknopfes 2 rutschen die Rastelemente 8 gegen den ersten O-Ring 42 und über diesen hinweg. Somit ist der Knopf 2 nach dem Aufsetzen gegen eine axiale Verschiebung und unbeabsichtigtes Herausfallen gesichert. Bei anderen in den Zeichnungen nicht dargestellten Ausführungsbeispielen kann das erreicht werden, wenn auf der Innenseite 54 des Eingriffknopfes 2 ein umkreisförmiges flexibles Element, ähnlich zu dem O-Ring 42 vorgesehen ist, um mit den überstehenden Rastelementen (dann an der Hülse 5) einen axialen Abziehschutz für den Eingriffknopf 2 zu bilden.

Der würfelförmige Eingriffkopf 36 gleitet dabei in die etwas Spiel aufweisende komplementäre Vierkant-Innenbohrung 55, so dass ein Formschluss hergestellt wird. Der Arzt stellt nun den Anfangsabstand ein. Dabei verdreht er den Knopf 2, um das gewünschte räumliche Verhältnis der die Knochen haltenden Stäbe zu erreichen. Es ist hervorzuheben, dass diese Einstellung nachfolgend fix ist. Durch die ineinander gesetzten verdrehsicheren Rohre 4 und 5 ist auch bei einer Torsionsbelastung eine unbeabsichtigte Verdrehung der Vorrichtung 1 ausgeschlossen. Durch die dreistufige longitudinale Verstellung mit den Elementen 4, 10 und 5 ist eine sichere Festlegung des gewünschten Abstandes gesichert, der nicht unabsichtlich verstellbar ist, insbesondere wenn der Arzt den Einstellknopf 2 entfernt. Der Einstellknopf 2 kann mit einer Aufhängeeinrichtung 9 versehen sein, die ein Loch zur Durchführung beispielsweise einer Kordel aufweist.

Die nun bestehende Einstellung kann durch einen Skalenwert auf dem Innenschaft 4 abgelesen werden. Durch das Gewinde 26/30 ist der Hub, also die Änderung des Abstandes der Klemmelemente 11 und 12, je Umdrehung oder Teil einer Umdrehung des Einstellknopfes 2 festgelegt. Somit kann der Patient nachfolgend entsprechende Einstellungen nach ärztlicher Anweisung selbst vornehmen. Dafür erhält er einen Einstellknopf 2. Wenn dieser nicht aufgesetzt ist, ist, wie oben gesagt, die Vorrichtung gegen unbeabsichtigte Einstellungsänderungen gesichert. Auf dem Einstellknopf 2 ist vorteilhafterweise eine einem Rastelement 8 zugeordnete Markierung vorgesehen, die gegenüber einer Laufflächennut 39 auf der Hülse 5 aufgesetzt wird. Somit kann der Einstellknopf 2 definiert aufgesetzt werden. Nach Aufsetzen des Einstellknopfes 2 kann der Patient eine definierte einfach nachvollziehbare Drehung des Knopfes 2 vollführen. Bei zwei gegenüberliegenden Rastelementen 8 und vier Laufflächennuten 39 verspürt der Patient nach Vollführen einer Drehung von 90 Grad ein Rasten. Bei entsprechendem feinen Gewinde können so in vordefiniertem zeitlichen Abstand Längenveränderungen der Knochen voneinander eingestellt werden. Beispielsweise kann der Patient angehalten sein, jeden zweiten Tag drei vollständige Drehungen zu vollführen, das heisst den Knopf zu drehen, bis er zwölfmal ein Einrasten der Elemente 8 und 39 verspürt hat.

Bei drei Rastelementen 8 und einer einzigen oder drei im Abstand von 120 Grad angeordneten Laufflächennuten 39 ergibt sich eine Auflösung der Drehung von 120 Grad. Wenn drei Rastelementen 8 zwei gegenüberliegende Laufflächennuten 39 zugewiesen sind, beträgt die Winkelauflösung 60 Grad. Die gleiche Auflösung ist mit zwei Rastelementen 8 wie im vorliegenden Ausführungsbeispiel und sechs Laufflächennuten 39 erreichbar. Weitere Möglichkeiten ersieht der Fachmann nach Bedarf. Nach dem Abziehen des Einstellknopfes 2 bestehen keine über den Schaft überstehenden Elemente, so dass eine Verstellung unwahrscheinlich ist. Zur Sicherung der Einstellung kann eine hohlzylindrische Schutzhülse über den Einstellknopf 2 auf die Hülse 5 aufgeschoben werden, die durch den ersten O-Ring 42 gehalten wird, dessen Rotation durch ein Rotieren einer solchen Hülse nicht das Einstellelement 10 zum Drehen bringt. Bei Kontrolluntersuchungen gestattet die auf dem Innenschaft 4 vorgesehene Skala ein Ablesen und somit eine Kontrolle der ausgeführten Drehungen, das heisst der durchgeführten Längenveränderung.

Die Materialwahl der Komponenten von Hülse 5 und Innenschaft 4, Einstellelement 10 sowie der weiteren Elemente kann jeweils Stahl sein, so dass sich Stahl zu Stahl Berührungsflächen ausbilden.

Die in den Zeichnungen dargestellten Ausführungsbeispiele beziehungsweise die in der vorliegenden Beschreibung erwähnten Merkmale von verschiedenen Ausführungsbeispielen sind nicht notwendigerweise als voneinander unabhängige Ausführungsformen zu verstehen. Vielmehr ist es möglich, dass jedes in einem der Ausführungsbeispiele beschriebene Merkmal mit einem oder mehreren beliebigen anderen Merkmalen aus anderen Ausführungsbeispielen kombiniert werden kann, so dass sich andere, nicht wörtlich oder unter Bezugnahme auf Zeichnungen beschriebene Ausführungsbeispiele ergeben, die im Rahmen der vorliegenden Ansprüche in den beanspruchten Schutzbereich fallen.

### Bezugszeichenliste:

- 1: externe Befestigungsvorrichtung
- 2: Einstellknopf
- 3: Achse
- 4: Innenschaft
- 5: Hülse
- 6: Bügel
- 7: Longitudinalachse
- 8: Rastelement
- 9: Aufhängeeinrichtung
- 10: Einstellelement
- 11: erstes Klemmelement
- 12: zweites externes Klemmelement
- 13: strichlinierte Lage
- 14: Hohlkörper
- 15: Bohrung
- 16: Schraube
- 17: Anschlagkante
- 18: Anschlagkante
- 19: Klemmeinrichtung
- 20: Öffnungen
- 21: Klemmelement
- 22: Bügel
- 23: Aussenmantel
- 24: Zylindermantel
- 25: Innenbohrung
- 26: Innengewinde
- 29: Klemmschraube
- 30: gewindeter Schaft
- 31: sich aufweitender Abschnitt
- 32: zylindrischer Abschnitt
- 33: Nut
- 34: zylindrischer Abschnitt
- 35: Nut
- 36: Eingriffknopf
- 37: Aufgleitkanten
- 38: Innennut
- 39: Laufflächennut
- 41: Klemmring
- 42: erster O-Ring
- 50: Umlaufnut
- 51: Griffbereich
- 52: halbkugeliger Knopf
- 53: zweiter O-Ring
- 54: erste Stufe Innenbohrung
- 55: zweite Stufe Innenbohrung / Innenvierkant
- 56: erste Stufe Bohrung
- 57: zweite Stufe Bohrung
- 58: oberer Bereich
- 59: quer verlaufende Nut

## Patentansprüche

1. Externe Befestigungsvorrichtung (1), insbesondere zum Vergrössern eines Abstandes zwischen Klemmelementen (11, 12) für einen externen Fixator, mit einem zentralen Innenschaft (4), mit einer äusseren hohlen Hülse (5), in der der Innenschaft (4) axial (7) beweglich ist, wobei ein Ende (6) des Innenschaftes (4) über das eine Ende der Hülse (5) hinausragt, und mit einem Einstellelement (10), das an dem anderen Ende der Hülse (5) vorgesehen ist und mit dem Innenschaft (4) und/oder der äusseren hohlen Hülse (5) verbunden ist, wobei durch eine Drehung des Einstellelementes (10) die axiale Position (7) des Innenschaftes (4) in Bezug auf die Hülse (5) einstellbar ist, wobei ein Einstellknopf (2) vorgesehen ist, der in einem Formschluss lösbar auf das Einstellelement (10) aufsetzbar ist und dabei der aufgesetzte Einstellknopf (2) mit mindestens einem Bereich der Hülse (5) in Eingriff bringbar ist, **dadurch gekennzeichnet, dass** Einstellknopf (2) und Hülse (5) über jeweils mindestens ein komplementäres Rastelement (39 und 8) verfügen, so dass durch Anzahl und Anordnung der komplementären Rastelemente (39 und 8) nach jeweils einem vordefinierten Drehwinkel des Einstellknopfes (2) eine Rastposition mit einer geänderten Länge erreichbar ist.

2. Vorrichtung nach Anspruch 1, **dadurch gekennzeichnet, dass** die Hülse (5) über eine oder mehrere, vorteilhafterweise in einem regelmässigen Winkelabstand voneinander angeordnete, Vertiefungen (39) oder Erhebungen als Rastelemente verfügt, und dass der Einstellknopf (2) über eine oder mehrere vorteilhafterweise in einem regelmässigen Winkelabstand voneinander angeordnete, Erhebungen (8) oder Vertiefungen als Rastelemente verfügt.

3. Vorrichtung nach Anspruch 1, **dadurch gekennzeichnet, dass** die Erhebungen (8) des Einstellknopfes (2) über die Unterseite einer axialen Ausnehmung (54) hinausragenden Köpfe (52) von durch Federkraft (53), insbesondere durch einen O-Ring, gehaltenen Elementen (8) gebildet sind.

4. Vorrichtung nach einem der Ansprüche 1 bis 3, **dadurch gekennzeichnet, dass** auf der Aussenseite der Hülse (5) oder auf der Innenseite (54) des Eingriffknopfes (2) ein umkreisförmiges flexibles Element (42) vorgesehen ist, um mit den überstehenden Rastelementen (8) einen axialen Abziehschutz für den Eingriffknopf (2) zu bilden.

5. Vorrichtung nach einem der Ansprüche 1 bis 4, **dadurch gekennzeichnet, dass** der Einstellknopf (2) mit einer Aufhängeeinrichtung (9), insbesondere einer Öse, versehen ist.

6. Vorrichtung nach einem der vorhergehenden Ansprüche, **dadurch gekennzeichnet, dass** das Einstellelement (10) einen Schaft mit einem Aussengewinde (30) aufweist, dass der Innenschaft (4) mindestens teilweise hohl (25) ist und an seinem hohlen Ende über ein Innengewinde (26) verfügt, in dass das besagte Aussengewinde (30) eingreift, dass Innenschaft (4) und Hülse (5) einen zueinander komplementären verdrehsicheren Querschnitt aufweisen, dass das Einstellelement (10) und die Hülse (5) über komplementäre nachgiebige Rastmittel (33, 38, 41) verfügen, um das mit dem Innenschaft (4) verbundene Einstellelement (10) und die Hülse (5) gegenüber einer direkten axialen Verschiebung zueinander zu verriegeln.

## Claims

1. An external fixation device (1), particularly for the extension of the distance between two clamping members (11, 12) for an external fixator comprising a central internal rod (4), and comprising an outer hollow housing (5), in which internal rod (4) is movable axially (7), whereby one end (6) of the internal rod (4) projects over the one end of the housing (5), and comprising an adjusting member (10), that is disposed at the other end of the housing (5) and that is connected with the internal rod (4) and/or the outer hollow housing (5), whereby the axial position (7) of the internal rod (4) can be adjusted in relation to the housing (5) by means of rotation of the adjusting member (10), wherein an adjusting knob (2) is disposed that can be detachably attached to the adjusting member (10) with an essentially positive fit, and wherein the attached adjusting knob (2) can be placed in contact with at least one area of the housing (5), **characterized in that** adjusting knob (2) and housing (5) each have at least one complementary stop member (39 and 8) so that a stop position with an altered length can be reached through the number and arrangement of the complementary stop members (39 and 8) according to a predefined angle of rotation of adjusting knob (2).

2. The device according to claim 1 wherein the housing (5) has one or a plurality of recesses (39) or embossments, advantageously disposed at a regular angular pitch, as stop members and wherein adjusting knob (2) has one or a plurality of embossments (8) or recesses, advantageously disposed at a regular angular pitch, as stop members.

3. The device according to claim 1 wherein the embossments (8) on adjusting knob (2) are heads (52) projecting over the underside of an axial recess (54), formed by members (8) held by elastic force (53), particularly by an O-ring.

4. The device according to one of claims 1 to 3 wherein, on the outside of the housing (5) or on the inside (54) of the adjusting knob (2), a circumferential flexible member (42) is disposed to form, with the above-mentioned stop members (8), an axial removal protection for the adjusting knob (2).

5. The device according to one of claims 1 to 4 wherein the adjusting knob (2) is provided with a hanging device (9), particularly a loop.

6. The device according to one of the preceding claims wherein the adjusting member (10) has a rod with an external thread (30), internal rod (4) is at least partially hollow (25) and has an internal thread (26) at its hollow end into which said external thread (30) penetrates, and wherein the internal rod (4) and housing (5) have a torsion-resistant cross-section cooperating with each other, the adjusting member (10) and the housing (5) have a complementary conforming stop member (33, 38, 41) to lock the adjusting member (10) connected with internal rod (4) and the housing (5) against direct axial displacement.

## Revendications

1. Dispositif de fixateur externe (1), en particulier pour agrandir la distance entre deux éléments de serrage (11, 12) pour un fixateur externe, comprenant une barre interne centrale (4) et comprenant une manche creuse externe (5), dans laquelle la barre interne (4) peut être déplacée axialement (7), où un bout (6) de la barre interne (4) s'étend au delà d'un bout de la manche (5) et comprenant un élément de réglage (10) qui est disposé à l'autre bout de la manche (5) et qui est connecté avec la barre interne (4) et/ou la manche creuse externe (5), où la position axiale (7) de la barre interne (4) peut être ajustée par rapport à la manche (5) par rotation de l'élément de réglage (10), où un bouton de réglage (2) est prévu qui peut être attaché d'une manière amovible à l'élément de réglage (10) avec un engagement essentiellement positif, et où le bouton de réglage attaché (2) peut être placé en contact avec au moins une zone de la manche (5), **caractérisé en ce que** le bouton de réglage (2) et la manche (5) possèdent chacun au moins un élément butoir complémentaire (39 et 8) pour que une position de butoir avec une longueur changée peut être utilisée par nombre et arrangement des éléments butoir complémentaires (39 et 8) selon un angle de rotation prédéfini du bouton de réglage (2).

2. Dispositif selon la revendication 1, où la manche (5) comprend un ou plusieurs creux (39) ou bosses, avantageusement disposés dans une distance angulaire régulière, en tant que éléments butoir, et où le bouton de réglage (2) comprend un ou plusieurs bosses ou creux, avantageusement disposés dans une distance angulaire régulière, en tant qu'éléments butoir.

3. Dispositif selon la revendication 1, où les bosses (8) sur le bouton de réglage (2) sont des têtes (52), s'étendant au delà de la face inférieure d'un creux axial (54), formé par les éléments (8), tenu par une force élastique (53), en particulier par un joint torique.

4. Dispositif selon l'une des revendications 1 à 3, où sur l'extérieur de la manche (5) ou à l'intérieur (54) du bouton de réglage (2), un élément flexible circonférenciel (42) est prévu pour former avec les éléments boutoir susmentionnés (8) une protection contre un enlèvement axial du bouton de réglage (2).

5. Dispositif selon l'un des revendications 1 à 4, où le bouton de réglage (2) est muni d'un élément de suspension (9), particulièrement une dragonne.

6. Dispositif selon l'une des revendications précédentes, où l'élément de réglage (10) possède une barre avec un filetage externe (30), où la barre interne (4) est au moins partiellement creuse (25) et possède, à son bout creux, un filetage interne (26) dans lequel le filetage externe (30) s'engage, et où la barre interne (4) et la manche (5) possèdent une section transversale résistante à des torsions, coopérant l'une avec l'autre, où l'élément de réglage (10) et la manche (5) comprennent des éléments butoir flexibles complémentaires (33, 38, 41) pour verrouiller l'élément de réglage (10) connecté avec la barre interne (4) et la manche (5) contre un déplacement axial direct.
